# EUROPEAN PATENT SPECIFICATION

(11) **EP 1 767 212 B1**
(45) Date of publication and mention of the grant of the patent: **20.07.2011**
(21) Application number: 05752366.4
(22) Date of filing: 30.05.2005
(51) Int. Cl.: A61K 36/87, A23L 1/30

(54) **PLANTS OF GENUS AMPELOPSIS AND EXTRACTS THEREOF FOR USE IN THE TREATMENT OF SLEEP DISORDERS**
PFLANZEN DER GATTUNG AMPELOPSIS UND DEREN EXTRAKTE ZUR BEHANDLUNG VON SCHLAFSTÖRUNGEN
UNE PLANTE DU GENRE AMPELOPSIS ET D'EXTRAITS DE CETTE PLANTE POUR LE TRAITEMENT DE L'INSOMNIE

(30) Priority: 04.06.2004 CN 200410048628
(43) Date of publication of application: 28.03.2007
(73) Proprietor: Bright Future Pharmaceutical Laboratories Limited, Yuen Long Industrial Estate, Yuen Long (CN)
(72) Inventor: CHAN, Hsiaochang, Hong Kong (CN); GOU, Yulin, Hong Kong (CN); ROWLANDS, Dewi, Kenneth, Taikoo Shing Hong Kong (CN); CHUNG, Yiu, Wa, Hong Kong (CN)
(74) Representative: Kramer - Barske - Schmidtchen
(86) International application number: PCT/CN2005/000749
(87) International publication number: WO 2005/117922

(56) References cited:
- CN-A- 1 375 289
- CN-A- 1 393 443
- CN-A- 1 442 136
- CN-A- 1 457 646
- CN-A- 1 457 774
- CN-C- 1 130 198
- CN-C- 1 142 155
- ZHOU T ET AL: "Isolation, structure determination and pharmacological activity of flavanonol from Ampelopsis grossedentata" ZHONGGUO YAOXUE ZAZHI - CHINESE PHARMACEUTICAL JOURNAL, GAI KAN BIAN-WEI-HUI, BEIJING, CN, vol. 31, no. 8, 1 January 1996 (1996-01-01), pages 458-461, XP009124783 ISSN: 1001-2494
- DU Q ET AL: "Purification of (+)-dihydromyricetin from leaves extract of Ampelopsis grossedentata using high-speed countercurrent chromatograph with scale-up triple columns" JOURNAL OF CHROMATOGRAPHY, ELSEVIER SCIENCE PUBLISHERS B.V. AMSTERDAM, NL, vol. 973, no. 1-2, 11 October 2002 (2002-10-11), pages 217-220, XP004382332 ISSN: 0021-9673
- "Amendment symposium in ZhengZhou" CELL BIOLOGY INTERNATIONAL, ACADEMIC PRESS, GB, vol. 29, no. 10, 1 October 2005 (2005-10-01), pages S1-S26, XP005086400 ISSN: 1065-6995
- DATABASE MEDLINE US NATIONAL LIBRARY OF MEDICINE (NLM), BETHESDA, MD, US; April 2002 (2002-04), WANG YAN; ZHOU LILING; LI RUI; WANG YING: "[Studies on the chemical constituents from Ampelopsis grossedentata]" XP002552507 Database accession no. NLM12583176
- DATABASE MEDLINE US NATIONAL LIBRARY OF MEDICINE (NLM), BETHESDA, MD, US; January 1997 (1997-01), CHEN Z; WANG M; CAI P; CHEN X: "[Determination of ampelopsin and myricetin in Ampelopsis cantoniensis]" XP002552508 Database accession no. NLM12572492
- DU Q ET AL: "Preparative separation of flavonoid glycosides in leaves extract of Ampelopsis grossedentata using high-speed counter-current chromatography" JOURNAL OF CHROMATOGRAPHY, ELSEVIER SCIENCE PUBLISHERS B.V. AMSTERDAM, NL, vol. 1040, no. 1, 18 June 2004 (2004-06-18), pages 147-149, XP004508928 ISSN: 0021-9673

## Description

Use of Ampelopsis plants and their extracts in the manufacture of a medicament for the treatment and prevention of sleep disorders.

### Technical Field

The present invention relates to the use of Ampelopsis plants and their extracts in the manufacture of a medicament for the treatment and prevention of sleep disorders.

### Background of the Invention

Insomnia is a common sleep disorder that requires adequate attention and treatment. Each year, 35-49 % of the adults suffer from insomnia. Man's sleep regulation center is located at the hypothalamus of the cerebral, where there are many hypothalamic neurotransmitters regulating the sleeping-awakening cycle (see: Mignot, E., Taheri, S., and Nishino, S., Sleeping with the hypothalamus: emerging therapeutic targets for sleep disorders. Nat. Neurosci. 2002, 5 Suppl: 1071-1075). The mechanism or means that affects or regulates sleeping involves many neurotransmitter systems, for instance, γ-aminobutyric acid, adenosine (purin), dopamine, hypocretins system (also known as orexins; phenyl dihydroquinazolin), monoaminergic system (for instance, 5-hydroxyptamine, noradrenalin and histamine).

Since drugs presently used for treating sleep disorders have different degrees of side effects, herbal medicines and other natural medicines that may help sleep are getting more and more attention. There are approximately 25 kinds of Ampelopsis plants around the world, including 17 in China, which are widely distributed in such provinces and autonomous regions as Guangxi, Guangdong, Yun'nan, Guizhou, Hu'nan, Hubei, Jiangxi and Fujian, etc. Except common medicine [Ampelopsis japonica (Thunb.) Makino], the majority of Ampelopsis plants are folk Chinese herbal medicines, most of which, Ampelopsis grossedentata (Hand-Mazz) W. T. Wang, [Ampelopsis cantoniensis (Hook.et Am.) Planch], Ampelopsis meliaefolia, Ampelopsis brevipedunculata (Uaxim.) Trautv, Ampelopsis megalophylla Diels et Gilg in particular, have functions such as promoting blood circulation, dissipating blood stasis, clearing heat and removing toxic substances, etc; Ampelopsis plants can prevent and cure icteric hepatitis, cold, wind heat, pharyngeal swelling and pain, wind-damp pain, calenture, getting drunken, etc., and pharmacological experiments have shown that main components of Ampelopsis plants have such functions as removing phlegm, reducing blood lipids, resisting inflammation, diuresis, and protecting the liver, etc.

Zhou et al., Chinese Pharmaceutical Journal, vol. 31, no. 8, 458-461, disclose the isolation, structure determination and pharmacological activity of flavanonol from Ampelopsis grossedentata.

Du et al., Journal of Chromatography A, 973 (2002), 217-220, disclose the purification of (+)-dihydromyricetin from leaves extract of Ampelopsis grossedentata using a high-speed countercurrent chromatograph with scale-up triple columns.

"Amendment symposium in ZhengZhou", Cell Biology International, vol. 29, no. 10, page S16, discloses a study on the characterization of new sedative compounds from an Ampelopsis extract using functional and genomic approaches.

DATABASE MEDLINE US NATIONAL LIBRARY OF MEDICINE, BETHESDA, MD, US, April 2002, Wang et al., discloses studies on the chemical constituents from Ampelopsis grossedentata.

DATABASE MEDLINE US NATIONAL LIBRARY OF MEDICINE, BETHESDA, MD, US, January 1997, Chen et al., discloses the determination of ampelopsin and myricetin in Ampelopsis cantoniensis.

Du et al., Journal of Chromatography A, 1040 (2004), 147-149, disclose the preparative separation of flavonoid glycosides in leaves extract of Ampelopsis grossedentata using high-speed counter-current chromatography.

### Summary of the Invention

The technical problem that the invention will tackle is to screen and study plants that have the function of treating sleep disorders and the parts of the plants that can treat sleep disorders. Since the mechanism or means that affects or regulates sleep involves many neurotransmitter systems, this invention screens and studies plant extracts that have synergetic effects on multiple mechanisms or means that affect or regulate sleep.

In order to solve the above problem, the invention provides the following technical proposal:
The use of Ampelopsis plants in the manufacture of a medicament for the treatment and prevention of sleep disorders.
The use of extracts of Ampelopsis plants in the manufacture of a medicament for the treatment and prevention of sleep disorders.

Said Ampelopsis plants include Ampelopsis grossedentata (Hand-Mazz) W. T. Wang, [Ampelopsis cantoniensis (Hook. et Am.) Planch], Ampelopsis meliaefolia, Ampelopsis brevipedunculata (Uaxim.) Trautv, Ampelopsis megalophylla Diels et Gilg, Ampelopsis bodinieri (Levl. et Vant.) Rehd, Ampelopsis brevipedunculat (Maxim.) Maxim ex Trautv, Ampelopsis brevipedunculat (Maxim.) Trautv. var. Hancei Rehd, Ampelopsis delavayana Planch, Ampelopsis japonica (Thunb.) Makino, Ampelopsis aconitifolia Bmge, Ampelopsis aconitifolia Bge. var. glabra Diels et Grig, Ampelopsis brevipedunculata (Maxim.) Trautv. var. kulingensisRehd, Ampelopsis humulifolia Bge, Ampelopsis heterophylla (Thunb.) Sieb. & Zucc, Ampelopsis chaffanjoni (Levl. et Vant.) Rehd.

According to said use, said Ampelopsis plants and/ or extracts of Ampelopsis plants can be mixed with drug carriers or food carriers.

Said extracts of Ampelopsis plants include extracts obtained through extraction of Ampelopsis plants with water, low alcohols, low ketone or acetic acid ethyl ester as solvents or random mixed solvents of these solvents; the low alcohols among the solvents used for extraction may be methanol, ethanol or n-butyl alcohol and the low ketones may be acetone; the extraction method may be self-immersion extraction, circumfluence extraction, ultrasonic extraction, circumfluence extraction and supercritical extraction. For instance, extract with the mixed solvent of ethanol:water (volume ratio: 90-10:10:90), preferably the mixed solvent of ethanol:water (volume ratio: 70:30), extract with acetone/water (volume ratio: 50:50) by means of circumfluence or extract directly by soaking with water.

Said extracts of Ampelopsis plants mainly contain protein, amino acid, amylose, inorganic nutrients, phenolic compounds and/or flavone, especially amino acid, amylose, phenolic compounds and/or flavone. Said extracts of Ampelopsis plants may mainly contain phenolic compounds and/or flavone.

To be specific, so-called 'treating sleep disorders' mentioned in this invention means that the drugs or healthcare products can help sleep and be used in daily life to alleviate or improve various sleep disorders due to different causes.

The dose of the plant of genus Ampelopsis of this invention, for instance, Ampelopsis grossedentata (Hand-Mazz) W. T. Wang, is 0.05-5000 mg/kg/day by weight and the preferred dose is 0.05-2500 mg/kg/day when preventing and curing sleep disorders. The dose of the extract of Ampelopsis plant of this invention, for instance, Ampelopsis grossedentata (Hand-Mazz) W. T. Wang, is 0.01-100 mg/kg/day by weight and the preferred dose is 0.01-50 mg/kg/day when used for preventing and curing sleep disorders. Due to different types and extents of sleep disorders of different individuals and individual differences (age, gender, etc.), the dose of plants and their extracts of this invention is not limited to the above scope.

The combination of this invention that prevents or cures sleep disorders is characterized as containing Ampelopsis plants and/or extracts or Ampelopsis plants. This invention includes healthcare products containing said combination that can prevent or cure sleep disorders, which are suitable for preventing and curing sleep disorders. This invention includes drugs containing said combination that can prevent or cure sleep disorders, which are suitable for preventing and curing sleep disorders.

Said Ampelopsis plants include Ampelopsis grossedentata (Hand-Mazz) W. T. Wang, [Ampelopsis cantoniensis (Hook. et Am.) Planch], Ampelopsis meliaefolia, Ampelopsis brevipedunculata (Uaxim.) Trautv, Ampelopsis megalophylla Diels et Gilg, Ampelopsis bodinieri (Levl. et Vant.) Rehd, Ampelopsis brevipedunculat (Maxim.) Maxim ex Trautv, Ampelopsis brevipedunculat (Maxim.) Trautv. var. Hancei Rehd, Ampelopsis delavayana Planch, Ampelopsis japonica (Thunb.) Makino, Ampelopsis aconitifolia Bmge, Ampelopsis aconitifolia Bge. var. glabra Diels et Grig, Ampelopsis brevipedunculata (Maxim.) Trautv. var. kulingensisRehd, Ampelopsis humulifolia Bge, Ampelopsis heterophylla (Thunb.) Sieb. & Zucc, Ampelopsis chaffanjoni (Levl. et Vant.) Rehd.

Extracts of Ampelopsis plants involved in this invention include extracts obtained through extraction of Ampelopsis plants with water, low alcohols, low ketones or acetic acid ethyl ester as solvents or random mixed solvents of these solvents; said low alcohols among the solvents used for extraction are methanol, ethanol or n-butyl alcohol and the low ketone is acetone; the extraction method is selected from self-immersion extraction, circumfluence extraction, ultrasonic extraction, circumfluence extraction and supercritical extraction. Extracts of Ampelopsis plants of this invention mainly contain protein, amino acid, amylose, inorganic nutrients, phenolic compounds and/or flavone, especially amino acid, amylose, phenolic compounds and/or flavone, or especially phenolic compounds and/or flavone.

When manufacturing a medicament that prevents or cures sleep disorders, the plant of genus Ampelopsis and their extracts of this invention may be added with various mineral substances, preferably mineral substances that play the role of supplementing necessary elements and trace elements that are apt to be insufficient in the body of organisms.

In addition, when manufacturing a medicament that prevents or cures sleep disorders, the plant of genus Ampelopsis and their extracts of this invention may be added with other natural drugs, amino acids and/or vitamins. Said natural drugs are free from special restrictions and shall preferably be Ganodorma lucidum, glutinous rehmannia, Chinese date, Schisandra chinensis (Turcz.) Baill, semen, ziziphi spinosae that are useful in stabilizing the spirit. In addition, lavender, Semen Biotae, radix polygalae, cortex albizziae, caulis polygoni multiflori, etc. that have sedative and relaxation effects may also be selected. There are no special requirements for the state of these plant drugs, which may be extract powder, essential oil extracted and herbal tea, etc. Also, there are no special requirements for the state of amino acid, which may be glutamine, troptophan, lysine, leucine, glutamic acid, isoleucine, and methionine, etc. And, there are no special requirements for vitamine, which, for instance, may be vitamine B6, vitamine B1, vitamine B2, folacin, vitamine C and vitamine E, etc.

When manufacturing a medicament that prevents or cures sleep disorders, the plant of genus Ampelopsiss and their extracts of this invention may be added with other elements, for instance, aloe, royal jelly, spirulina, gingko leaf, bifid bacterium, etc.

The plant of genus Ampelopsis and their extracts of this invention may be manufactured into different forms of drugs or healthcare products; said healthcare products of the invention include healthcare foods manufactured by adding the plant of genus Ampelopsis and their extracts of this invention into existing foods.

There are no special requirements for drugs or healthcare products of this invention, which may contain only Ampelopsis plants or their extracts, which may be in any of the following forms: solution, mixed suspended liquid, power, solid moulding, etc., may be tablets, capsules, powders, granules in dose form and be used in combined with other drugs.

### Description of the drawings.

Figure 1. Influence of Ampelopsis extracts on pentobarbital prolonged sleeping time in mice.
Figure 2. Influence of ECBRC-T3 concentration on relaxation effect of Guinea pig.

### Examples

### Example 1

Manufacturing method 1: 300 g of dry stem leaf (Ampelopsis grossedentata [Hand.-Mazz.] W. T. Wang), the stem leaf of which is known as tea vine (source: verified by Hou Yulin, the Chinese University of Hong Kong), was soaked in water and extracted 3 times using the circumfluence extraction method, 45 minutes each time. The extract was filtered whilst hot and subsequently evaporated to a small volume and dried to obtain 92 g of extracts (code: ECBRC-T1), representing a recovery rate of 30.7 %. Through determination, the extract contained 81.2 % of flavone, 1.1 % of polyphenol components as well as small quantities of amino acid and amylose.

Extraction method 2: 300 g of dry stem leaf (A. grossedentata [Hand.-Mazz.] W. T. Wang), was soaked in ethanol/water (V/V, 70:30) and extracted 3 times using the circumfluence method, 30 minutes each time. The extract was subsequently rotated and filtered whilst still hot (lower than 40°C) to remove ethanol, then freeze-dried to obtain 81 g of powder-like extract (code: ECBRC-T2), representing a recovery rate of 27 %. Through determination, the extract contained 90.3 % of flavone, 1.3 % of polyphenol components as well as small quantities of amino acid and amylose.

Manufacturing method 3: 100 g of extract of dry Ampelopsis plant (A. grossedentata [Hand.-Mazz.] W. T. Wang) was dissolved in 200 ml of acetone, heated and extracted using the circumfluence extraction method (45°C). The extract was subsequently rotated and filtered whilst still hot to approximately 20 ml, then diluted with 20-50 times of distilled water, kept static at 4°C to separate and allow sufficient crystal formation after approximately 12-24 hours. Approximately 39 g of crystals were recovered after drying (code: ECBRC-T3), representing a recovery rate of 39 %. Through determination, the extract contained 98 % of flavone as well as polyphenol components, amino acid and amylose.

Manufacturing method 4: 300 g of dry stem leaf (A. cantoniensis (Hook. et Arn.) Planch; verified by Hou Yulin from the Chinese University of Hongkong) was extracted with acetone/water (V/V, 50:50) using the circumfluence method. The extract was filtered whilst hot and subsequently evaporated to a small volume and dried to obtain 20 g of extracts (code: ECBRC-T4). Through determination, the extract contained 53.94 % of flavone as well as polyphenol, amino acids and amyloses.

Manufacturing method 5: 300 g of dry stem leaf (A. meliaefolia; verified by Hou Yulin from the Chinese University of Hong Kong) was soaked in methanol/water (V/V, 90:10), and extracted 3 times using the ultrasonic method, 60 minutes each time. The extract was filtered, rotated and then evaporated to remove the methanol, then dried to obtain 59 g of extract (ECBRC-T5). Through determination, the extract contained 14.8 % of flavone as well as polyphenol, amino acids and amylose.

Manufacturing method 6: 300 g of dry stem leaf (A. brevipedunculat (Maxim); verified by Hou Yulin from the Chinese University of Hongkong) was soaked in acetic acid ethyl ester and extracted using the countercurrent method for 2 hours. The extract was filtered, rotated and then evaporated to remove the solvents, giving the concentrated water a solution petroleum ether discoloration to get 36 g of extracts (ECBRC-T6). Through determination, the extract contained 46.1 % of flavone, 9.6 % of polyphenol contents as well as small quantities of amino acids and amyloses.

Manufacturing method 7: 300 g of dry stem leaf (A. megalophylla Diels et. Gilg; verified by Hou Yulin from the Chinese University of Hongkong), was soaked in n-butyl alcohol and extracted 4 times using the circumfluence extraction method. The extract was filtered whilst hot to remove the solvents, and subsequently treated with chloroform to get 44 g of extract (ECBRC-T7). Through determination, the extract contained 20.1 % of flavone, 22.3 % of polyphenol as well as amino acid and amylose.

Manufacturing method 8: preparation method of vine tea powder.

300 g of dry stem leaf (A. grossedentata [Hand.-Mazz.] W. T. Wang) was ground into 100 mesh of fine powder to be used.

### Example 2

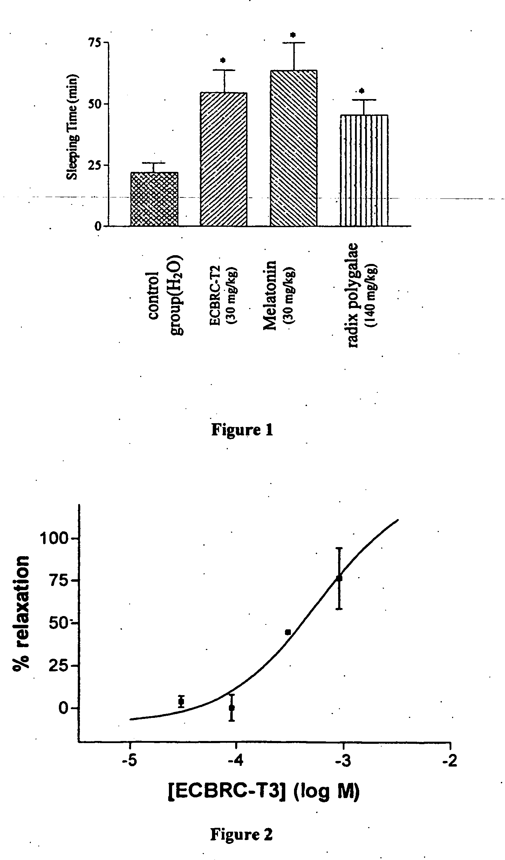

C57/B6 mice were randomised into groups and orally administered ECBRC-T2 (30 mg/kg), melatonin (30 mg/kg), Radix Valerianae (140 mg/kg) or vehicle (10 ml/kg H₂O), 30 min prior to low dose injection of sodium pentobarbital (30 mg/kg, i/p.). The animals were then placed on a heating pad (37°C) and their sleep latency to onset of sleep and the duration of sleep determined according to loss of righting reflex. Statistical analysis using one-way ANOVAR showed that each group to be significantly different (p<0.05, n>4) in sleep time when compared with the vehicle control group and the increase in sleep time, in the high-to-low order, was melatonin (Sigma, batch number: 12K1471), ECBRC-T2, Radix Valerianae (Roha Arzneimittel, batch number: 007420920). The result indicated that ECBRC-T2 had similar effect that helps sleep as melatonin (see figure 1).

### Example 3

C57/B6 mice were randomised into groups and orally administered ECBRC-T2 (30 mg/kg), melatonin (30 mg/kg), Radix Valerianae (140 mg/kg) or vehicle (10 ml/kg H₂O), 30 min prior to low dose injection of sodium pentobarbital (16 mg/kg, i/p.). The animals were then placed on a heating pad (37°C) and their sleep latency to onset of sleep and the duration of sleep determined according to loss of righting reflex. The experiment result showed that ECBRC-T2 had sedative /hypnotic effect (table 1)

**Table 1. Influence on mice sleep time of C57/B6 model.**

| Treatment | | | | |
|---|---|---|---|---|
| Group | Control group (H₂O) | ECBRC-T2 (30 mg/kg) | Melatonin (30 mg/kg) | Radix Valerianae (140 mg/kg) |
| Sleep time | 22 ± 4 | 54 ± 9* | 63 ± 11* | 45 ± 6* |
| (min±SEM) | n=6 | n=8 | n=4 | n=10 |

| | | | | |
|---|---|---|---|---|
| * p<0.05, compared with the control group. | | | | |

### Example 4

C57/B6 mice were randomised into groups and orally administered ECBRC-T1 (10, 50, 100, 500 mg/kg), 30 min prior to low dose injection of sodium pentobarbital (16 mg/kg, i/p.). The animals were then placed on a heating pad (37°C) and their sleep latency to onset of sleep and the duration of sleep determined according to loss of righting reflex. ECBRC-T1 (10, 50, 100, 500 mg/kg) 30 minutes later. The animals were placed on 37°C hot plates and the latency of sleep start and the duration of sleep were determined. The experiment result showed that ECBRC-T1 had the function of helping sleep and certain dosage-effect relationship (table 2).

**Table 2. Dosage-effect relationship of ECBRC-T1 for helping mice sleep of C57/B6 model.**

| Treatment | | | | | |
|---|---|---|---|---|---|
| Group | Control group (H₂O) | ECBRC-T1 (10 mg/kg) | ECBRC-T1 (50 mg/kg) | ECBRC-T1 (100 mg/kg) | ECBRC-T1 (500 mg/kg) |
| Sleep time | 18 ± 3 | 29 ± 5 | 37 ± 7*** | 38 ± 8*** | 36 ± 3*** |
| (min±SEM) | n=16 | n=9 | n=7 | n=7 | n=7 |

| | | | | | |
|---|---|---|---|---|---|
| ***p<0.001, compared with the control group. | | | | | |

### Example 5

C57/B6 mice were randomised into groups and orally administered with different dosages of ECBRC-T3 and melatonin (sigma, batch number: 12K1471), 30 min prior to low dose injection of sodium pentobarbital (16 mg/kg, i/p.). The animals were then placed on a heating pad (37°C) and their sleep latency to onset of sleep and the duration of sleep determined according to loss of righting reflex. The result showed that ECBRC-T3 could extend the sleep time of mice in a dosage-dependent manner and increased with the melatonin group synchronously (table 3).

**Table 3. Influence of ECBRC-T3 and melatonin on the mice sleep time of C57/B6 model.**

| Treatment | | |
|---|---|---|
| Dose | ECBRC-T3 | Melatonin |
| 10 mg/kg | 13 ± 2, n=5 | 31 ± 4*, n=4 |
| 20 mg/kg | 51 ± 2***, n=6 | 63 ± 5***, n=6 |
| 30 mg/kg | 47 ± 11*, n=6 | 68 ± 8***, n=6 |

| | | |
|---|---|---|
| * p<0.05, *** p < 0.001, both compared with the control group. | | |

### Example 6

### Remote sensing determination of mice activities.

Telemetric activity and temperature monitors were surgically implanted subcutaneously into male C57/B6 mice under ketamine (Alfasan, batch number: 112319) and xylazine (Alfasan, batch number: 073210) (75/10 mg/kg, i.p.) anaesthesia to remotely record activity. Correct placement was determined using X-ray radiography. Following a 2 week recovery and acclimatization period, the mice were orally treated with either test compounds, positive controls or vehicle; ECBRC-T3, zopiclone (Tocris, batch number: 1094) and An Shen Bu Nao Ye (Sedative and Brain-invigorating Fluid) (Jilin Aodong Yanbian Pharmaceutical Co., Ltd, batch number: 030659), respectively. Six hours following drug administration, the sleep time of mice of the ECBRC-T3 (20 mg/kg, p.o) group was significantly extended (table 4) as were mice treated with zopiclone (20 mg/kg, p.o group (table 5)), when compared to vehicle control. The result indicated that ECBRC-T3 (20 mg/kg, p.o.) could generate a similar degree of sedative-hypnotic effect as zopiclone (20 mg/kg, p.o.).

**Table 4. Comparison of hypnotic effect between ECBRC-T3 and An Shen Bu Nao Ye (Sedative and Brain-invigorating Fluid) 6 hours after the drugs were administered.**

| Drug and dose | Control group | ECBRC-T3 | An Shen Bu Nao Ye (Sedative and Brain-invigorating Fluid) |
|---|---|---|---|
| | H₂O | 20 mg/kg | 10 ml/kg |
| Telemetric Activity | 54.2 ± 3.6 | 65.4 ± 4.7* | 54.4 ± 2.5 |
| (% sleep time ± SEM) | n=7 | n=3 | n=5 |

| | | | |
|---|---|---|---|
| * p<0.05, compared with the distilled water control group. | | | |

**Table 5. Hypnotic effect of Zopiclone on the model mouse 6 hours after the drugs were administered.**

| Drugs and dose | Control group | Zopiclone |
|---|---|---|
| | 2 % EtOH | 20 mg/kg |
| Telemetric Activity | 51.9 ± 2.1 | 66.6 ± 5.1* |
| (% sleep time ± SEM) | n=4 | n=3 |

| | | |
|---|---|---|
| * p<0.05, compared with the 2% EtOH control group. | | |

### Example 7

### Isolated organ test

Guinea pig aorta was harvested from adult male guinea pigs following euthanisation via cervical dislocation. Aortic rings were suspended in carbonated Krebs solution (37°C) containing 3 µM indomethacin (to prevent endogenous prostacyclin production) at a resting tension of 1 g. Care was taken not to remove or damage the endothelial lining of the ring so as not disrupt any nitric oxide dependant relaxation. The tissue was then allowed to equilibrate for 1-2 hours, followed by pre-contraction with 40 mM KCI. After washout, ECBRC-T3 was assessed for contractility by adding directly to the aorta at resting tension, whereas relaxant effect was assessed by pre-contracting the aorta with phenylepherine (1 µM) followed cumulatively adding the ECBRC-T3 to the bath. Results demonstrated that ECBRC-T3 had no contractile effect on aortic smooth muscle. However, following pre-contraction with phenylepherine, ECBRC-AG-Act was able to concentration dependently relax guinea pig aorta with an effective concentration of approximately 600 µM (see figure 2).

### Example 8

### Sub-acute toxicity test

In accordance with OECD guidelines, half lethal dose is no longer regarded as an acceptable method for determining the toxicity of drugs. Rather, it is replaced by giving high dose in a single time or multiple doses within 24 hours. The experiments and results are given below:
SD rats (8 weeks of age, half female and half male), 5 rats per group, were administered vine tea extract (method 2, ECBRC-T2) (2000 mg/kg/24hrs) or distilled water (10 ml/kg/24hrs) by intragastic administration. Drugs were administered at a single dose then observed daily for 14 consecutive days for toxicity. Then, the euthanasia operation and the toxicological section inspection were performed. The result showed that compared with the control group, weight increase, food intake, skin/hair color and brightness of the rats showed no significant difference, indexes of its organs such as heart, lung, liver, spleen, genital organs, adrenal gland, pituitary gland etc. showed no significant difference, its hematological indicators such as white cell count, red cell count, platelet, hemoglobin and hematocrit, etc. showed no significant difference and its bio-chemical indicators such as total bilirubin, SGOT, SGPT, urea, creatinine, sodium ion, potassium ion, chloride ion and carbon dioxide, etc., also showed no significant differences.

The result indicated that when given vine tea extract (ECBRC-T2) 2000 mg/kg/24hrs as a high single oral dose SD rats had no any acute toxicity and death, so its half lethal dose was greater than 2000 mg/kg (approximately 200 times that of adult human dose).

### Example 9

### Phase I clinical test

A preliminary clinical test (phase I clinical test) was carried out in Zhengzhou University: 29 healthy volunteers, to whom insomnia had never occurred, were in good health during the testing period. And then, they were given a 3-day encephalogram test at from 11:30 pm each night to 6:30 am the next morning for 7 hours a day. Baseline test and environmental adaptability investigation were performed on the first night and placebo (200 mg of wheat starch) or 200 mg of vine tea extract (method 2, ECBRC-T2) were given on the second and third nights respectively. The study adopts double blind design to eliminate any influence of placebo or observation error. The average baseline value was used for matching t test, the result of which indicated that compared with placebo; the vine tea extract could significantly increase NREM sleeping time by 6 % while reducing sleep waiting time by 3 %. Considering wide variance in the baseline values of the recipient, although the analysis had statistical significance, it could not reflect the response of an individual recipient to drug treatment accurately. Further data analysis indicated that among all the samples, 91 % of recipient had positive response to drug treatment, namely, could either reduce sleep waiting time, or increased NREM sleeping time, or both. According to self-comparison and analysis, 71 % of the recipient improved their sleep waiting time, which was decreased by 53.8p4.7 % on average; 83 % of the recipient improved their NREM sleeping time, which increased by 24.2p6.1 % on average. This detailed analysis indicated that the vine tea extract had sedative/hypnotic effect over human body (see table 6).

NREMS in the table meant non-rapid eye movement sleep, also known as orthodox sleep or slow wave sleep, while REMS means rapid eye movement sleep, also known as paradoxical sleep or fast wave sleep.

**Table 6. Influence of ECBRC-T2 on sleeping time and sleep waiting time of healthy recipients.**

| % sleeping time ± SD | ECBRC-T2 | | | Placebo | | |
|---|---|---|---|---|---|---|
| NREMS | 68.1879 | ± 13.73* | n=29 | 62.7119 | ±21.1532 | n=29 |
| REMS | 21.3196 | ± 4.5269 | n=29 | 25.4171 | ±13.9314 | n=29 |
| Sleep waiting time | 6.3409 | ± 3.8619* | n=29 | 9.8409 | ±7.5709 | n=29 |

| | | | | | | |
|---|---|---|---|---|---|---|
| *p<0.05, compared with the placebo group. | | | | | | |

### Example 10

### Preliminary clinical test

4 people suffering from insomnia without other physiological diseases took 1 g of vine tea powder (example 1, manufacturing method 8) with hot water continuously for one month each night before going to bed. As a result, 4 patients were sleepy and could fall asleep within 30 minutes each day after taking the vine tea powder with extended sleeping time during the night and felt in good spirit on the next day. Statistical data indicated that the vine tea powder had the effect of helping sleep and increasing sound sleep over the human body.

## Claims

1. Use of the plant of genus Ampelopsis in the manufacture of a medicament for the treatment and prevention of sleep disorders.

2. Use of extracts of the plant of genus Ampelopsis in the manufacture of a medicament for the treatment and prevention of sleep disorders.

3. The use according to claim 1 or 2, wherein said the plant of genus Ampelopsis includes Ampelopsis grossedentata (Hand-Mazz) W. T.Wang, [Ampelopsis cantoniensis (Hook.et Am.) Planch], Ampelopsis meliaefolia, Ampelopsis brevipedunculata (Uaxim.) Trautv, Ampelopsis megalophylla Diels et Gilg, Ampelopsis bodinieri (Levl. et Vant.) Rehd, Ampelopsis brevipedunculat (Maxim.) Maxim ex Trautv, Ampelopsis brevipedunculat (Maxim.) Trautv. var. Hancei Rehd, Ampelopsis delavayana Planch, Ampelopsis japonica (Thunb.) Makino, Ampelopsis aconitifolia Brnge, Ampelopsis aconitifolia Bge. var. glabra Diels et Grig, Ampelopsis brevipedunculata (Maxim.) Trautv. var. kulingensisRehd, Ampelopsis humulifolia Bge, Ampelopsis heterophylla (Thunb.) Sieb. & Zucc, Ampelopsis chaffanjoni (Lev1. et Vant.) Rehd.

4. The use according to claim 1 or 2, wherein the plant of genus Ampelopsis can be mixed with drug carriers or food carriers.

5. The use according to claim 2, wherein the plant of genus Ampelopsis extract is that extracts obtained through extraction using water, low alcohol, low ketone or acetic acid ethyl ester as solvent or random mixed solvents of these solvents.

6. The use according to claim 5, wherein the low alcohol mentioned in the solvents for extraction is methanol, ethanol, n-butyl alcohol and the low ketone is acetone; the extraction method is selected from self-immersion extraction, circumfluence extraction, ultrasonic extraction, circumfluence extraction and supercritical extraction.

7. The use according to claim 5, wherein the plant of genus Ampelopsis extracts mainly contains protein, amino acid, amylose, inorganic nutrients, phenol compounds and/or flavone.

8. The use according to claim 7, wherein the plant of genus Ampelopsis extracts mainly contains amino acid, amylose, phenol compounds and/or flavone.

9. The use according to claim 8, wherein the plant of genus Ampelopsis extracts mainly contains phenol compounds and/or flavone.

10. A composition comprising the plant of genus Ampelopsis and/or their extracts for use in treating and preventing sleep disorders.

11. The composition for use according to claim 10, wherein the plant of genus Ampelopsis comprises Ampelopsis grossedentata, Ampelopsis meliaefolia, Ampelopsis brevipedunculata Trautv, Ampelopsis megalophylla Diels et Gilg, Ampelopsis bodinieri Rehd, Ampelopsis brevipedunculat Maxim ex Trautv, Ampelopsis brevipedunculat Trautv. var. Hancei Rehd, Ampelopsis delavayana Planch, Ampelopsis japonica Makino, Ampelopsis aconitifolia Brnge, Ampelopsis aconitifolia Bge. var. glabra Diels et Grig, Ampelopsis brevipedunculata Trautv. var. kulingensisRehd, Ampelopsis humulifolia Bge, Ampelopsis heterophylla Sieb. & Zucc, Ampelopsis chaffanjoni Rehd.

12. The composition for use according to claim 10, wherein the plant of genus Ampelopsis extract includes extracts obtained through extraction using water, low alcohol, low ketone or acetic acid ethyl ester as solvent or random mixed solvents of these solvents.

13. The composition for use according to claim 12, wherein the low alcohol mentioned in the solvents for extraction is methanol, ethanol, n-butyl alcohol and the low ketone (acetone); the extraction method is selected from self-immersion extraction, circumfluence extraction, ultrasonic extraction, circumfluence extraction and supercritical extraction.

14. The composition for use according to claim 10, wherein the plant of genus Ampelopsis extract mainly contains protein, amino acid, amylose, inorganic nutrients, phenol compounds and/or flavone.

15. The composition for use according to claim 14, wherein the plant of genus Ampelopsis extract mainly contains amino acid, amylose, phenol compounds and/or flavone.

16. The composition for use according to claim 15, wherein the plant of genus Ampelopsis extract mainly contains phenol compounds and/or flavones.

17. A healthcare product comprising the composition of the plant of genus Ampelopsis and/or their extracts according to claim 10 for use in treating and preventing sleep disorders.

18. A drug comprising the composition of the plant of genus Ampelopsis and/or their extracts according to claim 10 for use in treating and preventing sleep disorders.

## Patentansprüche

1. Verwendung der Pflanze der Gattung Ampelopsis bei der Herstellung eines Medikaments zur Behandlung und Prävention von Schlafstörungen.

2. Verwendung von Extrakten der Pflanze der Gattung Ampelopsis bei der Herstellung eines Medikaments zur Behandlung und Prävention von Schlafstörungen.

3. Verwendung nach Anspruch 1 oder 2, bei der die Pflanze der Gattung Ampelopsis Ampelopsis grossedentata (Hand-Mazz) W. T. Wang, [Ampelopsis cantoniensis (Hook.et Am.) Planch], Ampelopsis meliaefolia, Ampelopsis brevipedunculata (Uaxim.) Trautv, Ampelopsis megalophylla Diels et Gilg, Ampelopsis bodinieri (Levl. et Vant.) Rehd, Ampelopsis brevipedunculat (Maxim.) Maxim ex Trautv, Ampelopsis brevipedunculat (Maxim.) Trautv. var. Hancei Rehd, Ampelopsis delavayana Planch, Ampelopsis japonica (Thunb.) Makino, Ampelopsis aconitifolia Brnge, Ampelopsis aconitifolia Bge. var. glabra Diels et Grig, Ampelopsis brevipedunculata (Maxim.) Trautv. var. kulingensisRehd, Ampelopsis humulifolia Bge, Ampelopsis heterophylla (Thunb.) Sieb. & Zucc, Ampelopsis chaffanjoni (Levl. et Vant.) Rehd umfasst.

4. Verwendung nach Anspruch 1 oder 2, bei der die Pflanze der Gattung Ampelopsis mit Arzneistoffträgern oder Nahrungsmittelträgern gemischt werden kann.

5. Verwendung nach Anspruch 2, wobei es sich bei dem Extrakt der Pflanze der Gattung Ampelopsis um Extrakte handelt, die durch Extraktion unter Verwendung von Wasser, eines niedermolekularen Alkohols, eines niedermolekularen Ketons oder von Essigsäureethylester als Lösungsmittel oder beliebiger Mischlösungsmittel aus diesen Lösungsmitteln erhalten worden sind.

6. Verwendung nach Anspruch 5, bei welcher der genannte niedermolekulare Alkohol in den Lösungsmitteln zur Extraktion Methanol, Ethanol, n-Butylalkohol ist, und das niedermolekulare Keton Aceton ist, wobei das Extraktionsverfahren aus Selbsteintauchextraktion, Umlaufextraktion, Ultraschallextraktion, Umlaufextraktion und überkritischer Extraktion ausgewählt ist.

7. Verwendung nach Anspruch 5, bei welcher die Extrakte der Pflanze der Gattung Ampelopsis vorwiegend Protein, Aminosäure, Amylose, anorganische Nährstoffe, Phenolverbindungen und/oder Flavon enthalten.

8. Verwendung nach Anspruch 7, bei welcher die Extrakte der Pflanze der Gattung Ampelopsis vorwiegend Aminosäure, Amylose, Phenolverbindungen und/oder Flavon enthalten.

9. Verwendung nach Anspruch 8, bei welcher die Extrakte der Pflanze der Gattung Ampelopsis vorwiegend Phenolverbindungen und/oder Flavon enthalten.

10. Zusammensetzung, umfassend die Pflanze der Gattung Ampelopsis und/oder deren Extrakte zur Verwendung bei der Behandlung und Prävention von Schlafstörungen.

11. Zusammensetzung zur Verwendung nach Anspruch 10, bei der die Pflanze der Gattung Ampelopsis Ampelopsis grossedentata, Ampelopsis meliaefolia, Ampelopsis brevipedunculata Trautv, Ampelopsis megalophylla Diels et Gilg, Ampelopsis bodinieri Rehd, Ampelopsis brevipedunculat Maxim ex Trautv, Ampelopsis brevipedunculat Trautv. var. Hancei Rehd, Ampelopsis delavayana Planch, Ampelopsis japonica Makino, Ampelopsis aconitifolia Brnge, Ampelopsis aconitifolia Bge. var. glabra Diels et Grig, Ampelopsis brevipedunculata Trautv. var. kulingensisRehd, Ampelopsis humulifolia Bge, Ampelopsis heterophylla Sieb. & Zucc, Ampelopsis chaffanjoni Rehd umfasst.

12. Zusammensetzung zur Verwendung nach Anspruch 10, bei welcher der Extrakt der Pflanze der Gattung Ampelopsis Extrakte umfasst, die durch Extraktion unter Verwendung von Wasser, eines niedermolekularen Alkohols, eines niedermolekularen Ketons oder von Essigsäureethylester als Lösungsmittel oder beliebiger Mischlösungsmittel aus diesen Lösungsmitteln erhalten worden ist.

13. Zusammensetzung zur Verwendung nach Anspruch 12, bei welcher der genannte niedermolekulare Alkohol in den Lösungsmitteln zur Extraktion Methanol, Ethanol, n-Butylalkohol ist, und das niedermolekulare Keton Aceton ist, wobei das Extraktionsverfahren aus Selbsteintauchextraktion, Umlaufextraktion, Ultraschallextraktion, Umlaufextraktion und überkritischer Extraktion ausgewählt ist.

14. Zusammensetzung zur Verwendung nach Anspruch 10, bei welcher der Extrakt der Pflanze der Gattung Ampelopsis vorwiegend Protein, Aminosäure, Amylose, anorganische Nährstoffe, Phenolverbindungen und/oder Flavon enthält.

15. Zusammensetzung zur Verwendung nach Anspruch 14, bei welcher der Extrakt der Pflanze der Gattung Ampelopsis vorwiegend Aminosäure, Amylose, Phenolverbindungen und/oder Flavon enthält.

16. Zusammensetzung zur Verwendung nach Anspruch 15, bei welcher der Extrakt der Pflanze der Gattung Ampelopsis vorwiegend Phenolverbindungen und/oder Flavone enthält.

17. Gesundheitspflegeprodukt, umfassend die Zusammensetzung der Pflanze der Gattung Ampelopsis und/oder deren Extrakte nach Anspruch 10 zur Verwendung bei der Behandlung und Prävention von Schlafstörungen.

18. Arzneistoff, umfassend die Zusammensetzung der Pflanze der Gattung Ampelopsis und/oder deren Extrakte nach Anspruch 10 zur Verwendung bei der Behandlung und Prävention von Schlafstörungen.

## Revendications

1. Utilisation de la plante du genre Ampelopsis dans la fabrication d'un médicament pour le traitement et la prévention des troubles du sommeil.

2. Utilisation d'extraits de la plante du genre Ampelopsis dans la fabrication d'un médicament pour le traitement et la prévention des troubles du sommeil.

3. Utilisation selon la revendication 1 ou 2, dans laquelle ladite plante du genre Ampelopsis comprend les espèces Ampelopsis grossedentata (Hand-Mazz) W. T.Wang, [Ampelopsis cantoniensis (Hook. et Am.) Planch], Ampelopsis meliaefolia, Ampelopsis brevipedunculata (Uaxim.) Trautv, Ampelopsis megalophylla Diels et Gilg, Ampelopsis bodinieri (Levl. et Vant.) Rehd, Ampelopsis brevipedunculat (Maxim.) Maxim ex Trautv, Ampelopsis brevipedunculat (Maxim.) Trautv. var. Hancei Rehd, Ampelopsis delavayana Planch, Ampelopsis japonica (Thunb.) Makino, Ampelopsis aconitifolia Brnge, Ampelopsis aconitifolia Bge. var. glabra Diels et Grig, Ampelopsis brevipedunculata (Maxim.) Trautv. var. kulingensisRehd, Ampelopsis humulifolia Bge, Ampelopsis heterophylla (Thunb.) Sieb. & Zucc, Ampelopsis chaffanjoni (Levi. et Vant.) Rehd.

4. Utilisation selon la revendication 1 ou 2, dans laquelle la plante du genre Ampelopsis peut être mélangée à des véhicules pour médicaments ou à des véhicules pour aliments.

5. Utilisation selon la revendication 2, dans laquelle l'extrait de plante du genre Ampelopsis consiste en extraits obtenus par extraction avec de l'eau, un alcool inférieur, une cétone inférieure ou l'ester éthylique de l'acide acétique comme solvant, ou des solvants mélangés de manière aléatoire de ces solvants.

6. Utilisation selon la revendication 5, dans laquelle l'alcool inférieur mentionné parmi les solvants pour l'extraction est le méthanol, l'éthanol, l'alcool de n-butyle et la cétone inférieure est l'acétone ; le procédé d'extraction est choisi parmi l'extraction en auto-immersion, l'extraction de circumfluence, l'extraction sous ultrasons, l'extraction de circumfluence et l'extraction supercritique.

7. Utilisation selon la revendication 5, dans laquelle l'extrait de la plante du genre Ampelopsis contient essentiellement des composés de type protéine, acide aminé, amylose, nutriments inorganiques, phénol et/ou flavone.

8. Utilisation selon la revendication 7, dans laquelle l'extrait de la plante du genre Ampelopsis contient essentiellement des composés de type acide aminé, amylose, phénol et/ou flavone.

9. Utilisation selon la revendication 8, dans laquelle l'extrait de la plante du genre Ampelopsis contient essentiellement des composés de phénol et/ou de flavone.

10. Composition comprenant la plante du genre Ampelopsis et/ou ses extraits pour une utilisation dans le traitement et la prévention des troubles du sommeil.

11. Composition pour une utilisation selon la revendication 10, dans laquelle la plante du genre Ampelopsis comprend les espèces Ampelopsis grossedentata, Ampelopsis meliaefolia, Ampelopsis brevipedunculata Trautv, Ampelopsis megalophylla Diels et Gilg, Ampelopsis bodinieri Rehd, Ampelopsis brevipedunculat Maxim ex Trautv, Ampelopsis brevipedunculat Trautv. var. Hancei Rehd, Ampelopsis delavayana Planch, Ampelopsis japonica Makino, Ampelopsis aconitifolia Brnge, Ampelopsis aconitifolia Bge. var. glabra Diels et Grig, Ampelopsis brevipedunculata Trautv. var. kulingensisRehd, Ampelopsis humulifolia Bge, Ampelopsis heterophylla Sieb. & Zucc et Ampelopsis chaffanjoni Rehd.

12. Composition pour une utilisation selon la revendication 10, dans laquelle l'extrait de la plante du genre Ampelopsis comprend des extraits obtenus par extraction avec de l'eau, un alcool inférieur, une cétone inférieure ou l'ester éthylique de l'acide acétique comme solvant, ou des solvants mélangés de manière aléatoire de ces solvants.

13. Utilisation selon la revendication 12, dans laquelle l'alcool inférieur mentionné parmi les solvants pour l'extraction est le méthanol, l'éthanol, l'alcool de n-butyle et la cétone inférieure est l'acétone ; le procédé d'extraction est choisi parmi l'extraction en auto-immersion, l'extraction de circumfluence, l'extraction sous ultrasons, l'extraction de circumfluence et l'extraction supercritique.

14. Composition pour une utilisation selon la revendication 10, dans laquelle l'extrait de la plante du genre Ampelopsis contient essentiellement des composés de type protéine, acide aminé, amylose, nutriments inorganiques, phénol et/ou flavone.

15. Composition pour une utilisation selon la revendication 14, dans laquelle l'extrait de la plante du genre Ampelopsis contient essentiellement des composés de type, acide aminé, amylose, phénol et/ou flavone.

16. Composition pour une utilisation selon la revendication 15, dans laquelle l'extrait de la plante du genre Ampelopsis contient essentiellement des composés de phénol et/ou de flavone.

17. Produit de santé comprenant la composition de la plante du genre Ampelopsis et/ou ses extraits selon la revendication 10, pour une utilisation dans le traitement et la prévention des troubles du sommeil.

18. Médicament comprenant la composition de la plante du genre Ampelopsis et/ou ses extraits selon la revendication 10, pour une utilisation dans le traitement et la prévention des troubles du sommeil.
